(19) Europäisches Patentamt
European Patent Office
Office européen des brevets

(11) Veröffentlichungsnummer: **0 074 571**
**A1**

# (12) EUROPÄISCHE PATENTANMELDUNG

(21) Anmeldenummer: **82108093.4**

(22) Anmeldetag: **02.09.82**

(51) Int. Cl.³: **C 07 C 47/55**
C 07 C 45/63, C 07 B 9/00

(30) Priorität: **15.09.81 DE 3136561**

(43) Veröffentlichungstag der Anmeldung:
**23.03.83 Patentblatt 83/12**

(84) Benannte Vertragsstaaten:
**AT BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Marzolph, Gerhard, Dr.**
**Roggendorfstrasse 65**
**D-5000 Köln 80(DE)**

(72) Erfinder: **Blank, Heinz Ulrich, Dr.**
**Am Geusfelde 35**
**D-5068 Odenthal(DE)**

## (54) Verfahren zur Herstellung von para-substituierten 3-Brombenzaldehyden.

(57) Die vorliegende Erfindung betrifft ein Verfahren zur Herstellung para-substituierter 3-Brombenzaldehyde der Formel I

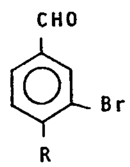

1

in der
R für Alkyl, oder Halogen, steht, indem man Benzaldehyde in einem inerten Lösungsmittel in Gegenwart eines Katalysators mit Brom in molaren Verhältnis 1 : 0,5 bis 1 : 0,6 und Chlor umsetzt, wobei das Verhältnis Brom zu Chlor 1 : 1 bis 1 : 1,3 beträgt.

EP 0 074 571 A1

0074571

BAYER AKTIENGESELLSCHAFT 5090 Leverkusen, Bayerwerk
14.09.81
Zentralbereich
Patente, Marken und Lizenzen Rt/Kü-c

# Verfahren zur Herstellung von para-substituierten 3-Brombenzaldehyden

Die vorliegende Erfindung betrifft ein Verfahren zur Herstellung von para-substituierten 3-Brombenzaldehyden durch Umsetzen von para-substituierten Benzaldehyden mit Brom und Chlor in Gegenwart eines Katalysators.

Die Meta-Bromierung von Benzaldehyden ist eine Reaktion, die oft nur mit unbefriedigenden Ausbeuten verläuft.

So beschreibt J. Org. Chem. 1958, 23, 1412 - 19 den Einsatz von Brom unter Verwendung von 2,5 Mol $AlCl_3$/Mol Aldehyd in einem lösungsmittelfreien Verfahren. Bei Einsatz von Benzaldehyd erhält man 3-Brombenzaldehyd in 52 % Ausbeute, aus dem p-substituierten 4-Methylbenzaldehyd wird das 3-Brom-Derivat in nur 44 % Ausbeute erhalten.

Durch Zusatz eines Lösungsmittels nach Org. Prep. Proc. Int. 6 (5), 251 - 3 (1974) kann die Ausbeute an 3-Brombenzaldehyd auf 75 % gesteigert werden. Der 3-Brom-4-methylbenzaldehyd wird in der geringen Ausbeute von 63 % erhalten. Die DE-OS 2 933 979 beschreibt die Bromierung von 4-Fluorbenzaldehyd zu 3-Brom-4-fluorbenzaldehyd in 57,1 % Ausbeute.

Die DE-OS 2 624 410 beschreibt den Einsatz von Brom und Chlor bei der Bromierung von Benzaldehyd. Um bei diesem Verfahren optimale Ausbeute zu erhalten, muß mit partiellem Umsatz des Benzaldehyds gearbeitet werden.

So erhält man bei 89 % Umsatz den 3-Brombenzaldehyd in 85 % Ausbeute, bei 93 % Umsatz nur noch in 79 % Ausbeute.

Es war also zu erwarten gewesen, daß die Bromierung von Benzaldehyden bei partiellem Umsatz durchgeführt werden sollte und daß die Bromierung para-substituierte Benzaldehyde schlechter verläuft als die von Benzaldehyd.

Die Erfindung betrifft ein Verfahren zur Herstellung para-substituierter 3-Brombenzaldehyde der Formel (I)

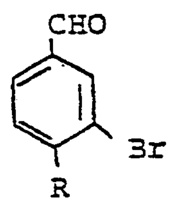

(I)

in der

R für Alkyl oder Halogen, steht

indem man Benzaldehyde der Formel II

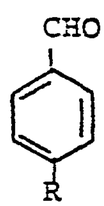

(II)

in der

R die oben angegebene Bedeutung besitzt,

in einem inerten Lösungsmittel mit Brom in molarem Verhältnis 1 : 0,5 bis 1 : 0,6 und Chlor in Gegenwart eines Katalysators umsetzt, wobei das Verhältnis Brom zu Chlor 1 : 1 bis 1 : 1,3 beträgt.

Das erfindungsgemäße Verfahren bietet neben den bisher nicht erreichbaren Ausbeuten an wertvollen Benzaldehyden, den Vorteil eines geringen Katalysatorbedarfs und einer einfachen Isolierung der Reaktionsprodukte, da die aufwendige destillative Abtrennung von nicht umgesetztem Ausgangsaldehyd entfällt.

Als Verbindungen der Formel II sind diejenigen bevorzugt in denen R für Halogen , wie Fluor, Brom, Chlor, Jod, insbesondere Fluor steht. 4-Fluorbenzaldehyd sei bevorzugt genannt.

Die Verbindungen der Formel II sind bekannt bzw. lassen sich nach bekannten Verfahren herstellen.

Als Lösungsmittel werden chlorierte Kohlenwasserstoffe wie z.B. Dichlormethan, Trichlormethan, Tetrachlormethan, 1,2-Dichlorethan, 1,2,3-Trichlorpropan oder Chlorbenzol eingesetzt.

Als Katalysator werden Lewis-Säuren wie z.B. Aluminiumchlorid oder Eisen-III-chlorid eingesetzt.

Die stöchiometrischen Verhältnisse zwischen Aldehyd und Katalysator sind 1 : 1,0 bis 1 : 1,5, vorzugsweise 1 : 1,2 bis 1 : 1,4, wobei hohe Katalysatormengen zu

einer schnelleren Reaktion führen.

Das Molverhältnis von Aldehyd zu Brom beträgt 1 : 0,5 bis 1 : 0,6, vorzugsweise 1 : 0,55.

Das Gemisch aus Brom und Chlor enthält beide Halogene im stöchiometrischen Verhältnis von Brom zu Chlor wie 1 : 1,0 bis 1 : 1,3, bevorzugt wie 1 : 1,1 bis 1 : 1,2. Man arbeitet vorzugsweise mit einem Überschuß an Chlor, um einen hohen Umsetzungsgrad des Aldehyds zu gewährleisten.

Das Gemisch aus Brom und Chlor kann auf verschiedene Weise hergestellt werden. So kann beispielsweise das Gemisch aus Katalysator, Aldehyd und Brom im Lösungsmittel vorgelegt werden und das Chlor gasförmig oder im Lösungsmittel gelöst zudosiert werden.

Gleichfalls ist es möglich Brom und Chlor gleichzeitig der Mischung aus Katalysator, Aldehyd und Lösungsmittel zuzugeben. Dabei muß gewährleistet sein, daß sich die gesammte Brommenge im Reaktionsgefäß befindet, bevor der Überschuß an Chlor hinzugegeben wird. Dies kann z.B. in der Art geschehen, daß 0 - 20 % der notwendigen Brommenge mit dem Katalysator und dem Aldehyd im Lösungsmittel vorgelegt werden und der Rest an Brom zusammen mit der äquimolaren Menge an Chlor gasförmig oder gelöst zudosiert wird und anschließend der für den gewünschten Umsatz notwendige Chlorüberschuß eingeleitet wird.



BAD ORIGINAL

Die Reaktionstemperatur liegt unterhalb des Siedepunktes des Lösungsmittels, bevorzugt zwischen 10 und 50 °C, besonders bevorzugt zwischen 15 und 30 °C.

Die erfindungsgemäß herstellbaren para-substituierten 3-Brombenzaldehyde sind wertvolle Zwischenprodukte für die Synthese insektizid wirksamer Pyrethroide (DE-OS 2 933 978).

Beispiel 1

3-Brom-4-fluorbenzaldehyd

252 g (2 Mol) 98,1 %iger 4-Fluorbenzaldehyd wird bei 20° zu der Suspension von 330 g (2,4 Mol) wasserfreiem Aluminiumchlorid in 300 ml Methylenchlorid getropft und 0,5 h bei 20°C gerührt. Man gibt 176 g (1,1 Mol) Brom dazu und leitet während 2 Stunden bei 20 bis 25°C 85,2 g (1,2 Mol) Chlor in die Reaktionsmischung. Man rührt bis zum Ende der Gasentwicklung und gibt auf Eis/Wasser. Die organische Phase wird abgetrennt, getrocknet und eingedampft. Der Rückstand wird bei 120°C/26 mbar destilliert. Man erhält 377,3 g 3-Brom-4-fluorbenzaldehyd (Gehalt: 98,5 %ig). Ausbeute: 91,5 %.

Beispiel 2

3-Brom-4-chlorbenzaldehyd

Zu der Suspension von 173,4 g (1,3 Mol) Aluminiumchlorid in 200 ml Dichlorethan tropft man die Lösung von 140 g (1 Mol) 4-Chlorbenzaldehyd in Dichlorethan und rührt 0,5 h bei 20°C. Dann tropft man die gekühlte Lösung aus 88 g (0,55 Mol) Brom und 35,5 g (0,5 Mol) Chlor in 200 ml Dichlorethan in 2h zu, rührt bis zum Ende der Gasentwicklung und tropft anschließend die Lösung von 10,6 g (0,15 Mol) Chlor in Dichlorethan zu. Nach beendeter Gasentwicklung wird auf Eis/Wasser gegeben, die organische Phase

abgetrennt und destilliert. Man erhält bei 150°C/24 mbar 201,4 g 3-Brom-4-Chlorbenzaldehyd (Gehalt: 97 %ig)
Ausbeute: 88,8 %.

Beispiel 3:
3-Brom-4-methylbenzaldehyd
Zu der vorliegenden Suspension von 173,4 g (1,3 Mol) wasserfreiem Aluminiumchlorid in 300 ml Methylenchlorid tropft man 120 g (1 Mol) 4-Methylbenzaldehyd bei 20°C zu und rührt eine Stunde nach. Anschließend tropft man die gekühlte Lösung von 88 g (0,55 Mol) Brom und 39,0 g (0,55 Mol) Chlor in 200 ml Methylenchlorid während drei Stunden unter die Oberfläche der Reaktionsmischung zu. Nach Abklingen der Gasentwicklung gießt man die Mischung auf 1 kg Eis/Wasser-Mischung, trennt die organische Phase ab, trocknet mit $MgSO_4$ und destilliert das Methylenchlorid ab. Der Rückstand besteht aus 98,1 %igem 3-Brom-4-methylbenzaldehyd vom Schmelzpunkt: 45-46°C.
Ausbeute: 175 g ≙ 86,3 % der Theorie.



BAD ORIGINAL

Patentansprüche

1. Verfahren zur Herstellung para-substituierter 3-Brombenzaldehyde der Formel I

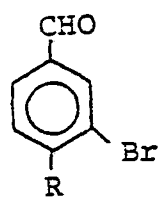

(I)

in der

R für Alkyl oder Halogen steht,

indem man Benzaldehyde der Formel II

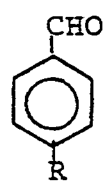

(II)

in der

R die oben angegebene Bedeutung besitzt,

in einem inerten Lösungsmittel in Gegenwart eines Katalysators mit Brom im molaren Verhältnis 1 : 0,5 bis 1 : 0,6 und Chlor umsetzt, wobei das Verhältnis Brom zu Chlor 1 : 1 bis 1 : 1,3 beträgt.

2. Verfahren nach Anspruch 1, dadurch gekennzeichnet, daß man als Verbindung der Formel II 4-Fluorbenzaldehyd einsetzt.

3. Verfahren nach Anspruch 1, dadurch gekennzeichnet, daß das Verhältnis Brom zu Chlor wie 1 : 1,1 bis 1 : 1,2 ist.

4. Verfahren nach Anspruch 1, dadurch gekennzeichnet, daß das Verhältnis Aldehyd zu Brom 1 : 0,55 ist.

5. Verfahren nach Anspruch 1, dadurch gekennzeichnet, daß der Katalysator Aluminiumchlorid ist.



BAD ORIGINAL

Europäisches Patentamt

# EUROPÄISCHER RECHERCHENBERICHT

0074571
Nummer der Anmeldung

EP 82 10 8093.4

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| X,D | DE - A1 - 2 624 410 (SHELL INTERNATIONALE RESEARCH MAATSCHAPPIJ)<br>* ganzes Dokument *<br>-- | 1-5 | C 07 C 47/55<br>C 07 C 45/63<br>C 07 B 9/00 |
| A | EP - A2 - 0 034 741 (RIEDEL DE HAEN)<br>* Beispiel 2 *<br>-- | 1 | |
| A,D | DE - A1 - 2 933 979 (BAYER)<br>* Beispiel 4 *<br>---- | 1,5 | |

RECHERCHIERTE SACHGEBIETE (Int. Cl.³)

C 07 B 9/00
C 07 C 45/63
C 07 C 47/55

KATEGORIE DER GENANNTEN DOKUMENTE

X: von besonderer Bedeutung allein betrachtet
Y: von besonderer Bedeutung in Verbindung mit einer anderen Veroffentlichung derselben Kategorie
A: technologischer Hintergrund
O: nichtschriftliche Offenbarung
P: Zwischenliteratur
T: der Erfindung zugrunde liegende Theorien oder Grundsatze
E: alteres Patentdokument, das jedoch erst am oder nach dem Anmeldedatum veroffentlicht worden ist
D: in der Anmeldung angefuhrtes Dokument
L: aus andern Grunden angefuhrtes Dokument
&: Mitglied der gleichen Patentfamilie, ubereinstimmendes Dokument

X Der vorliegende Recherchenbericht wurde fur alle Patentanspruche erstellt

| Recherchenort | Abschlußdatum der Recherche | Prufer |
|---|---|---|
| Berlin | 09-12-1982 | KNAACK |